# EUROPEAN PATENT SPECIFICATION

(11) **EP 1 009 316 B1**
(45) Date of publication and mention of the grant of the patent: **09.10.2013**
(21) Application number: 98938429.2
(22) Date of filing: 06.08.1998
(51) Int. Cl.: A61F 11/00

(54) **APPARATUS AND METHOD FOR AN AUDITORY STIMULATOR**
VORRICHTUNG UND METHODE ZUR GEHÖRSTIMULATION
DISPOSITIF ET PROCEDE POUR STIMULER L'AUDITION

(30) Priority: 07.08.1997 US 54942 P
(43) Date of publication of application: 21.06.2000
(73) Proprietor: Bauman, Natan, Cheshire, CT 06410 (US)
(72) Inventor: BAUMAN, Natan, Cheshire, CT 06410 (US); JUNEAU, Roger, P., Destrehan, LA 70047 (US)
(74) Representative: Somnier, Jean-Louis
(86) International application number: PCT/US1998/016412
(87) International publication number: WO 1999/007302

(56) References cited:
- WO-A1-90/03089
- GB-A- 2 134 689
- US-A- 3 764 748
- US-A- 4 222 393
- US-A- 5 167 236
- US-A- 5 403 262

## Description

### BACKGROUND OF THE INVENTION

### 1. Field of the Invention

The present invention relates to an Auditory Pathway Stimulator for management through central habituation of patients suffering from Tinnitus or Hyperacusis, or both. More particularly, the present invention relates to a system and method for reducing the debilitating effects of tinnitus by means of an all-in-the-canal, open-ear, noise stimulus of the auditory pathway.

### 2. General Background of the Invention

Tinnitus is a wide spread auditory symptom affecting approximately 17% of the general population growing to 33 % in the elderly. This percentage translates to about 44 million people in the United States. Most who experience tinnitus manage well, in that it is not disruptive in daily life. However, approximately 25% of people experiencing tinnitus are encumbered to the extent that they actively seek help. The above statistics correspond to approximately 10 million sufferers in the United States and 2 million of these sufferers are debilitated by tinnitus to the point of total disruption of the quality of life (Jastreboff P.J., Gray W.C., and Gold S.L.: "Neurophysiological Approach to Tinnitus Patients", The American Journal of Otology, 17:236-340 ©1996). In fact, some extreme cases have led to suicide. At present, there is no cure for tinnitus. Most sufferers are told by medical and audiological professionals they should "learn to live with it."

Over 300 research projects funded by public and private agencies have been conducted to address tinnitus and its harmful effects during the last two decades. A cure is still unforeseen in the near future. This invention is an important step to a systematic solution with long-term significant results.

The concept of tinnitus is difficult to convey since it is a "Phantom Auditory Perception" representing lack of certain neuro activity in the auditory system rather than an increased activity. It is also a symptom associated with many auditory and non auditory pathologies.

Unmanageable tinnitus is due to a conditioned reflex between the central auditory pathways and the limbic system. In short, when the auditory brainstem detects a new sound such as tinnitus it automatically assigns high priority to the new auditory signal. This detection in turn provokes a negative emotional association in the limbic system producing a very strong conditioned reflex perceived as a thread which activates the body's fight-or-flight response and since this is an internally generated sound one can not control its volume or remove themselves from it.

In the case of tinnitus, once the new pattern of auditory neural activity is detected and locked into the limbic system the tinnitus becomes extremely prominent. Basic neuroscience predicts that it is relatively easy to reverse this signal recognition process by exposing the patient to low levels of broad band noise for a long period of time. This broad band noise will interfere with the tinnitus recognition process making it more difficult to separate the tinnitus signal from background neuronal activities and, since there are no longer other processes increasing recognition of this intruding signal the tinnitus becomes no longer detected.

In recent years masking of tinnitus using various noise signals of adjustable width and amplitude have been developed as a result of scientific research done by Jack Vernon. These devices yielded very limited success. More recently, a neurophysiological model of tinnitus management has been developed by Pawel Jastreboff. Using specific protocol, attempts were made to habituate patients to the presence of tinnitus. Habituation infers training patients not to be aware of tinnitus except when deliberately focusing their attention on it. Even then, perceived tinnitus should no longer induce annoyance or irritation.

It has been shown that the introduction of a noise signal can help in the therapy. Behind-the-ear noise generators have yielded an 83 percent success rate. Yet the units remain big, cumbersome and cosmetically unappealing.

Manufacturers of hearing aids are regulated by the United States Food and Drug Administration (FDA) as a medical device establishment. Hearing aid components used in the assembly of custom open-ear tinnitus habituators and produced as a combined hearing aid/tinnitus habituator device or as a stand-alone tinnitus device will be categorized, not as a Class I hearing device, but rather as a Class III prosthetic device under 21 C.F.R. § 874.3400 entitled "Tinnitus masker". The tinnitus habituator differs from the tinnitus masker in that it does not emit a signal intended to overpower the tinnitus head/ear noise, but to stimulate the auditory pathways with a broad-band low-level signal. Its intent, however, is to aid in tinnitus and FDA has decided the device described in this application will fall into the Class III classification. FDA regulates medical devices based on safety and effectiveness. The hearing aid components used in the construction of the tinnitus habituator have been used in the assembly of millions of Class I hearing aids and are therefore known to be safe when used as directed. The efficacy is the issue, in that there is no established protocol by FDA in the management of tinnitus. The Class III classification stems from the absence of this protocol. The Class I level would be more cost-efficient to produce and in turn reduce the cost to the public. It is the protocol that teaches the patient to focus on the signal of the tinnitus habituator and not focus on the tinnitus head noise which enables the tinnitus device though central auditory habituation to reduce the debilitating effects of tinnitus or hyperacusis. Tinnitus habituators without a well designed therapy would not be significantly effective. Also, masking tinnitus via a traditional masker is counter productive (Gold S.L., Gray W.C., Hu S., and Jastreboff P.W. "Selection and Fitting of Noise Generators and Hearing Aids for Tinnitus Patients", Proceedings of the Fifth International Tinnitus Seminar 1995 edited by Reich G.E. and Vernon J.A. ©American Tinnitus Association, Portland OR USA 1996 p.312-314; Jastreboff P.J. and Hazel J.W.P. "A Neurophysiological Approach to Tinnitus: Clinical Implications" British Journal of Audiology, 1993. 27. 7-17). It is very important to avoid masking tinnitus. In fact, there are reports of patients having long term "masking" therapy who realized no relief from the perception of the tinnitus or the annoyance resulting from the tinnitus. After entering into habituation therapy, both the perception of and the annoyance from tinnitus were reduced (Jastreboff P.J., Gray W.C., and Gold S.L.: "Neurophysiological Approach to Tinnitus Patients", The American Journal of Otology, 17:236-340 ©1996).

Products designed to be used to fill the room with a low level noise, not FDA regulated, have proven to offer minimum effectiveness, because sound reaching the ear drum is unstable and inconsistent due to head shadow effect, occlusion to the external ear canal, and the Doppler effect caused by motion of the patient's head, such as turning back and forth vigorously. Additionally, the size of this unit makes it difficult to transport and impractical to use outside of the home since the noise would distract others.

U.S. Patent No. 5,403,262 issued to Gooch describes a circuit means of random noise generation, which is digitally processed to selected center frequencies, bandwidth and intensity. A random noise signal is transduced to acoustical energy by a speaker or headphone. Other means of delivering the masking signal to the patient's ear may be by way of using a low-power transmitter or electromagnetic coil to deliver the signal from the tinnitus masker to a receiver worn in or on the sufferer's ear. Gooch's circuit is intended for room noise generation to mask tinnitus, not habituate it, as a patient goes to sleep.

U.S. Patent No. 5,325,872, issued to Westerham et al., discloses an invention which relates to a Tinnitus masker with one or more signal generators, a controllable amplifier, transducers and comprises means for generating a continuously repeated, sinusoidal pure-tone signal which continuously and slowly moves through an audiofrequency range in a cyclic manner to remove tinnitus for a period of time. Westerham et al. predicates its design of the masker on the reasoning that "tinnitus is very frequently perceived as a tone". The described embodiment is a behind-the-ear (B-T-E) device.

U.S. Patent No. 4,222,393 issued to Hocks et al. describes a Tinnitus masker device whereby the patient is subjected to external sounds of different pitch, one right after another, to enable the patient to identify the particular external sound having the same pitch as the subjective tinnitus sound perceived by the patient. The patient is then provided a power-operated sound generator producing a range of frequencies extending above and below the perceived pitch to mask the tinnitus sound. The tinnitus masker may be worn in the same manner as a hearing aid or combined with one. Once again, the device is a masker intended to immerse the tinnitus head noise below audibility. This is accomplished by using a noise signal with a patient-selected center frequency where the output noise signal is of broader band-width and intensity than the tinnitus therefore facilitating a masking effect of the tinnitus. The actual noise generation of the masker is by a noise generator. It can only be produced in a behind-the-ear instrument.

U.S. Patent No.5,167,236 issued to Junker describes a behind-the-ear Tinnitus-masker which is an electronic circuit designed so that the sound spectrum produced through an ear-piece contains a line spectrum with a fundamental tone.

U.S. Patent No. 4,870,688 issued to Voroba et al. describes a hearing aid comprising an electronic circuit designed using a pseudorandom frequency noise generator as a sound source to be connected to a hearing aid circuit for use in patients with "ringing" or "buzzing" in the head, commonly called tinnitus. The drawings in the patent do not define the object and only identify a standard hearing aid microphone as the source.

Other patents that may be of interest include the following:
U.S. Patent No. 5,024,612, External ear canal pressure regulating device and tinnitus suppression device;
U.S. Pat No. 5,628,330, Apparatus for treating people afflicted with tinnitus;
U.S. Patent No. 4,735,968, Method of treating tinnitus with AOAA;
U.S. Patent No. 5,279,292, Charging system for implantable hearing aids and tinnitus maskers;
U.S. Patent No. 5,064,858, Protected complex of procaine for the treatment of symptoms from narcotics addiction, tinnitus and Alzheimer's disease;
U.S. Patent No. 4,954,486, Furosemide as tinnitus suppressant;
U.S. Patent No. 4,984,579, Apparatus for treatment of sensorineural hearing loss, vertigo, tinnitus and aural fullness;
U.S. Patent No. 4,956,391, Protected complex of procaine for the treatment of symptoms from narcotics addiction, tinnitus and Alzheimer's disease;
U.S. Patent No. 5,589,183, Method and apparatus for treatment of neurogenic diabetes mellitus, and other conditions;
U.S. Patent No. 4,759,070, Patient controlled master hearing aid;
U.S. Patent No. 4,226,248, Phonocephalographic device;
U.S. Patent No. 5,563,140, Use of 1-(aminoalkyl)-3-(benzyl)-quinoxaline-2-one derivatives for the preparation of neuroprotective compositions;
U.S. Patent No. 4,680,798, Audio signal processing circuit for use in a hearing aid and method for operating same;
U.S. Patent No. 3,764,748, Implanted hearing aids;

Whether the failure of current tinnitus devices is due to the incomplete or inaccurate clinical knowledge or whether the failure is existing product performance, the end result is tinnitus and hyperacusis patients are experiencing an inadequate quality of life.

### BRIEF SUMMARY OF THE INVENTION

The apparatus of the present invention solves the problems confronted in the art in a simple and straightforward manner.

Unlike Gooch, the present invention is completely self-contained in the ear shell and does not use electromagnetic or transmitter technology. Further, it does not incorporate digital technology for band-width and center-frequency selection. The present invention performs optimally in open-ear design. In many cases the stimulator can be combined with a hearing aid circuit to compensate for a hearing loss.

Unlike Hocks et al., the present invention embodies an open-ear in-the-canal device without insertion loss and with better cosmetics. Further, noise generation is by thermal circuit noise, requiring fewer electronic components and better product reliability.

Unlike Voroba et al., the sound source may be a thermal noise specifically designed for band width, gain and low current drain optimized for hearing aid batteries. It is not a pseudorandom frequency noise generator.

The sound generator of an embodiment of the present invention provides sound of stable temporal characteristics, such that head movement does not cause a change in the signal at the TM of the wearer of the present invention.

The sound generator of an embodiment of the present invention provides a wide band sound spectrum to emulate real world sound so there is not a strong negative association with a new pattern sound in the central auditory system.

The present invention is a cosmetically superior design as compared to a BTE with the earmold and connecting tube assembly. This feature is genuinely beneficial since the perception of tinnitus sound or any sound can be habituated only when the stigma of wearing the device does not evoke any form of negative emotional response.

The present invention allows enhanced telephone use with the product of the present invention in place.

The current invention does not have moisture problems as those associated with BTE's in the earmold tubing; the current invention eliminates the need for earmold tubing.

The current design has a matched acoustic response for both ears since the earmold and earmold tubing are eliminated.

The current design yields a better angle of attack for the sound spectrum to impinge the TM as compared to the BTE whose receiver is housed behind the ear and must travel through earmold tubing and the earmold. Variances in the tubing length and earmold insertion depth of BTE's affect the spectrum greatly.

The current design is less susceptible to wax impaction since the receiver is located in the superior external ear canal quadrant.

The device according to an embodiment of the present invention has a volume control taper which allows very discrete adjustment of low level sounds while maintaining a very stable signal output.

### BRIEF DESCRIPTION OF THE SEVERAL VIEWS OF THE DRAWINGS

For a further understanding of the nature, objects, and advantages of the present invention, reference should be had to the following detailed description, read in conjunction with the following drawings, wherein like reference numerals denote like elements and wherein:
FIG. 1 shows a canal open-ear device in a human ear;
FIG. 2 shows a Helix open-ear device in a human ear;
FIG. 3 shows a CIC open-ear device in a human ear;
FIG. 4 shows the CIC open-ear device with an alternative retention system to hold the device in place in the human ear;
FIG. 5 shows the device graphically illustrated via magnetic resonance imaging (MRI) in a typical human ear as viewed from the top of the head, superior to inferior.
FIG. 6 shows a preferred embodiment of a Class A electrical circuit, in which the volume control is accomplished with a 100K ohm variable resistor in which minimum resistance yields maximum volume;
FIG. 7 shows a preferred embodiment of a two-stage Class A electrical circuit;
FIG. 8 shows a preferred embodiment of a three-stage Class D electrical circuit;
FIG. 9 shows a preferred embodiment of a programmable K-amp three-stage Class D electrical circuit;
FIG. 10 shows a preferred embodiment of a programmable Intrigue™ three-stage Class D electrical circuit;
FIG. 11 shows the frequency response of various volume control positions of the TRI; and
FIGS. 12A-12E show frequency spectrums of the noise signal with a 1.5V battery, wherein "SPL" is root-mean-square noise sound pressure level generated by the device of the present invention, with Fig. 12A showing a maximum level of 82.1 dB SPL, with Fig. 12B showing a maximum level of 82.7 dB SPL, with Fig. 12C showing a maximum level of 82.9 dB SPL, with Fig. 12D showing a maximum level of 85.5 dB SPL, with Fig. 12E showing a maximum level of 86.4 dB SPL.

### DETAILED DESCRIPTION OF THE INVENTION

During development, as the open ear auditory pathway stimulator, to manage tinnitus and hyperacusis, was nearing completion, it was referred to as a Tinnitus Retraining Instrument (TRI). This describes the associated therapy more than the device but segregates it from other devices, such as maskers.

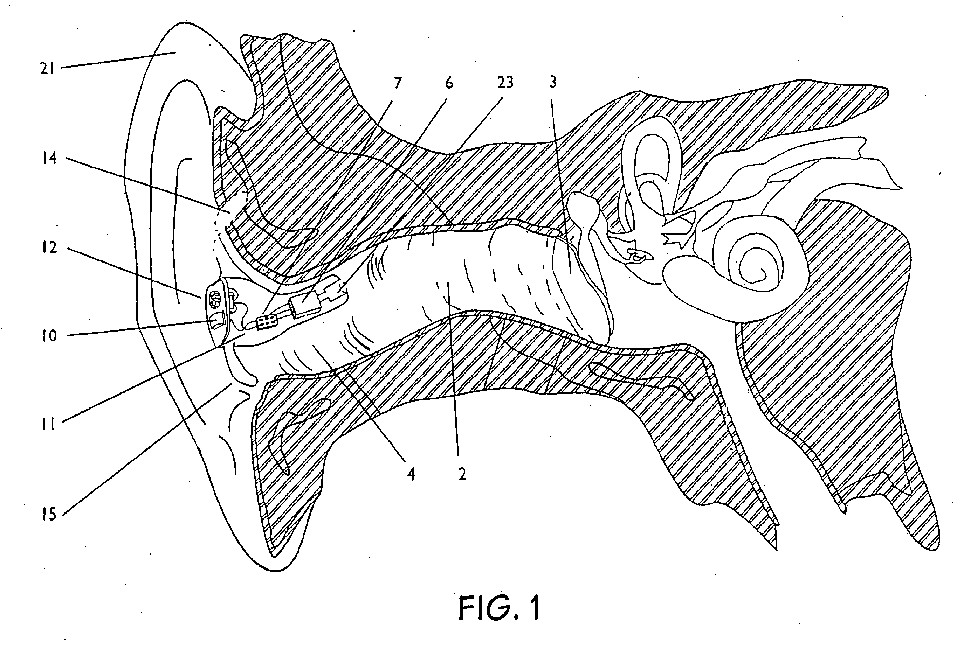

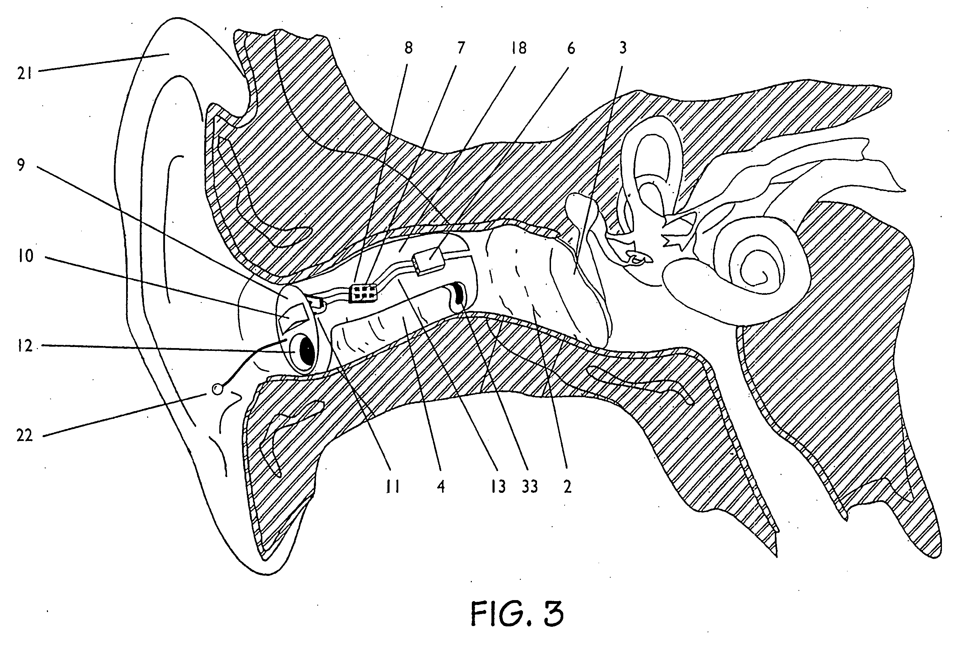

Figure 1 shows one configuration of a complete TRI device 1 in the human ear from a side perspective. Figure 1 shows a canal open-ear model 1 made in an acrylic shell. The tympanic membrane, TM, or ear drum 3 is deflected by sound waves emitting from the receiver 6 of the TRI 1. Two sources of sound reach the TM 3. One, sound is delivered by the TRI. The other sounds arrive from surrounding ambient sources, such as another human voice. The construction of the TRI 1 is such that the external ear canal 2 is completely open in the inferior portion or bottom half 4. This is important to assure delivery of normal outside sounds, without introducing intermodulation that would alter the original signal. If the external ear canal is occluded, typically low frequencies are introduced, making the wearer report that their voice sounds like it is in a barrel. If the external ear canal 2 is sufficiently open (approximately 50 percent), the ear canal resonance is unchanged and the wearer reports normal sound quality. The same performance would be accomplished if the superior ear canal were left open, although wax-related problems with the receiver 6 make it a less-than-optimum design in terms of product reliability. The retention arms 14 and 15 position the shell housing of the TRI 1 to maintain the openness of the external ear canal 2 and properly aim the receiver at the superior portion of the TM 3, its most sensitive surface area. The electronic circuit is mounted on the faceplate 9 (see Fig. 3) with a battery door 10 and battery contacts 11 connecting power via litz wire 13 to the noise source 8 then to the amplifier 7 and on to the receiver 6, where noise is generated and directed through an acoustic horn 23 to the TM 3. The level of transmitted noise is adjusted by the volume control 12. The canal-open-ear (COE) design is cosmetically a superior design as compared to a behind-the-ear (BTE) with the earmold and tubing connection which fits over the external ear (pina) 21. This is genuinely beneficial, since the perception of tinnitus sound can be habituated only when the stigma of wearing the device does not evoke any form of an emotional response associated with wearing the device.

One could use a Microtronic #6 volume control adapter ring and wire 13 could be insulated stranded wire similar to Litz 44 gauge 5 strand magnet wire.

With the present invention, there is enhanced telephone use with the product in place, in terms of sound transmission and the placement of a phone receiver adjacent the external ear canal, as in usual use. The BTE product must be repositioned with the telephone receiver if the BTE is also a hearing aid or removed if the BTE is not equipped with a microphone. Additionally, the present invention has a matched acoustic response for both ears since the earmold and earmold tubing associated with BTE's are eliminated. The current design yields a better angle of attack for the sound spectrum to impinge the TM 3 as compared to the BTE whose receiver is housed behind the ear and must travel through earmold tubing and the earmold. Variances in the tubing length and earmold insertion depth affect the spectrum greatly. The present invention has a sound envelope of stable temporal characteristics, where head movement will not cause a change in the signal at the TM 3.

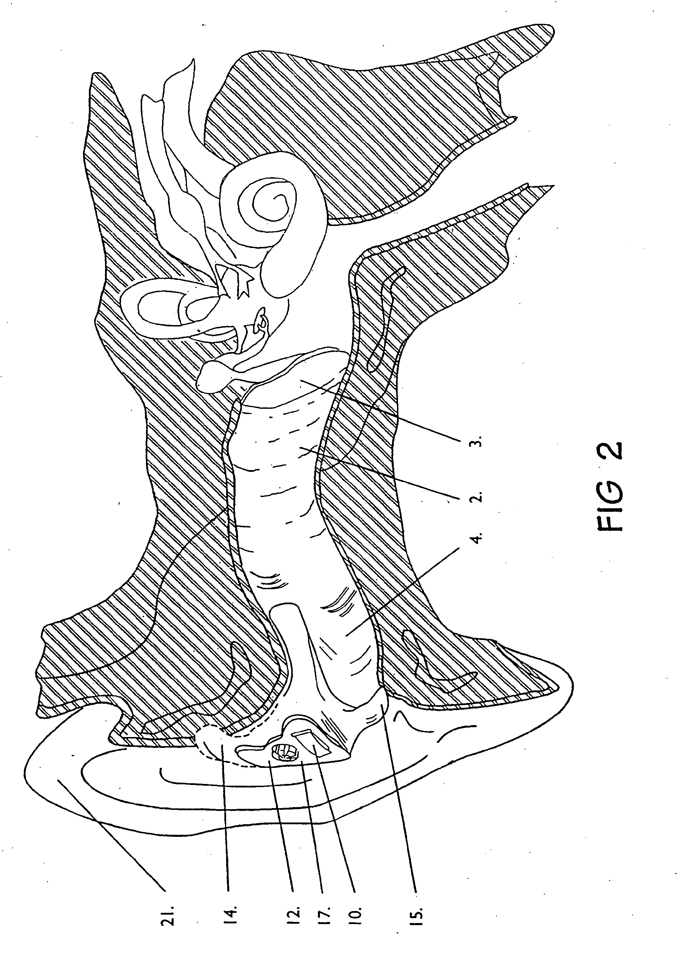

Figure 2 shows an open ear, helix based TRI 17. It is used to fit ears which are too small for the COE or CIC units by filling the concha bowl in the helix of the pina 21. The theory of operation is as above. A larger battery can also be used, increasing service life between battery changes.

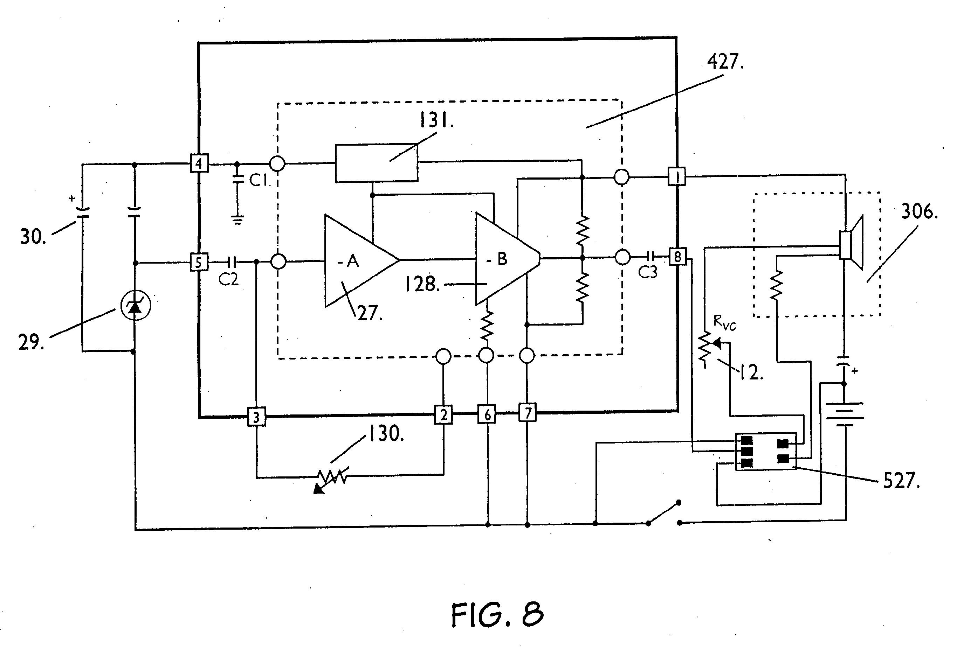

Figure 3 shows an open ear, completely-in-the-canal (CIC) based TRI 18. The CIC TRI 18 uses a unique positioning system to properly aim the receiver 6 at the TM 3. As in Figure 1, this positioning is important to deliver a sound envelope of stable temporal characteristics, where head movement will not cause a change in the signal at the TM 3. The sagital plane or face plate 9 is designed to have an opening at the apex of the external ear canal 2, then to leave the inferior portion 4 of the ear canal free and unobstructed medially until the distal, or medial end of the device where a medial ipsolateral-route-of-signal (IROS) vent retainer 33 supports the device 18 upward into the superior portion of the external ear canal 2 and at the same time keeping the inferior canal 4 open and veritably unobstructed for free-field sound transmission. The CIC is the product of choice to offer the best cosmetic solution and has a built in extraction cord 22 since the device resides deeply in the external ear canal 2. This extraction cord 22 also serves as a volume control extension which allows adjustment of the noise level by rolling the nubbin on the cord between the index finger and thumb. The CIC design preferably uses a battery size "5 A" and should use an efficient circuit, such as the class D (Fig. 8), to have a reasonable battery life.

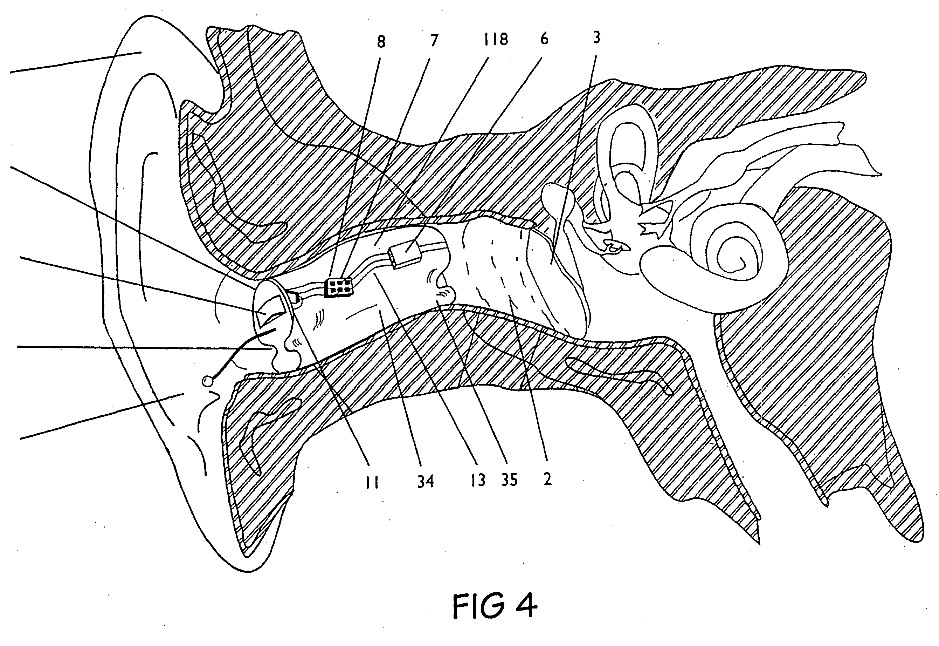

Figure 4 shows an alternative retention design for an open ear CIC 118; all theory of operation remains the same.

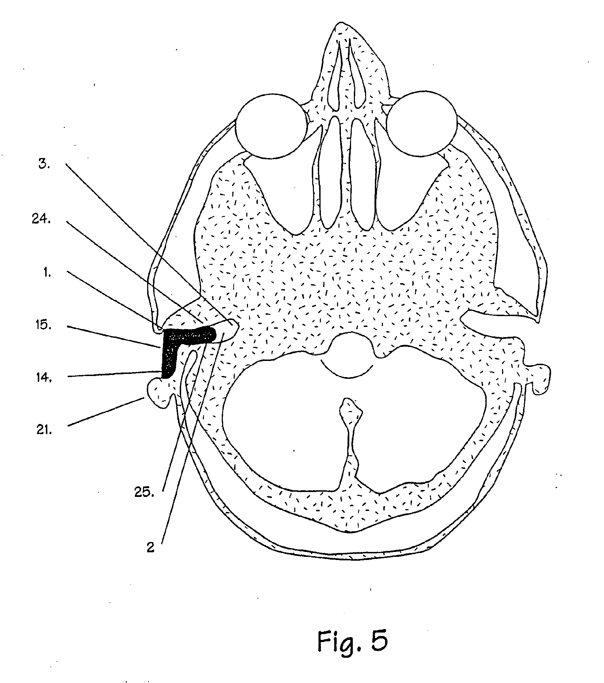

Figure 5 shows a COE based TRI device graphically illustrated via magnetic resonance imaging (MRI) in a typical human ear as viewed from the top of the head, superior to inferior. The external ear canal 2 and its first and second directional bend are exhibited. The anterior 24 and posterior 25 planes are shown to demonstrate how the COE shell must be molded to faithfully follow the ear canal. The helix 14 and cavum 15 retention arms maintain the spatial relationships to fill the superior canal while the inferior canal 4 is left open, at the same time the anterior 24 and posterior 25 canal quadrants are filled, aiming the receiver at the superior portion of the TM 3.

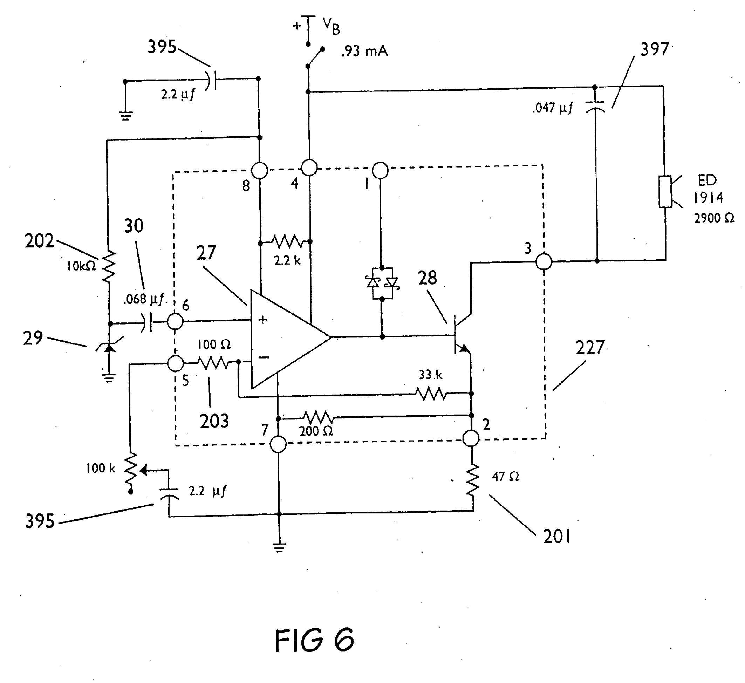

FIG. 6 shows a preferred embodiment of a Class A electrical circuit. A basic operational amplifier 27 feeds into a transistor 28 forming the basic circuit. A Zener diode 29, Motorola type MLL4684 or equivalent, is set between ground potential and input into a capacitor 30 which establishes the corner frequency of the amplifier circuit. The noise generator formed by the Zener diode 29 and the RC input circuit requires very little current drain which is primarily dictated by the requirements of the OpAmp/Transistor. The transducers are selected based on frequency response and impedance. Higher impedance transducers reduce the current draw. The total current drain required for the device is governed by the electrical / acoustic efficiency of the transducer and how much sound volume the device is set to deliver. The volume control 12 taper is selected to have as much "bend" in it as possible. The reason is that most of the control occurs in the first few thousands of Ohms of resistance. So if the resistance selected on the volume control is minimum, gain is maximized to the loudest noise output. Resistance taper of the volume control yields about one and a half dB increments with a range typically of twenty-four dB. In Figure 6, the transducer is a model ED 1914 commercially available from Knowles Electronics, Chicago, IL, and V_{B} is a battery which can last typically about 60 hours.

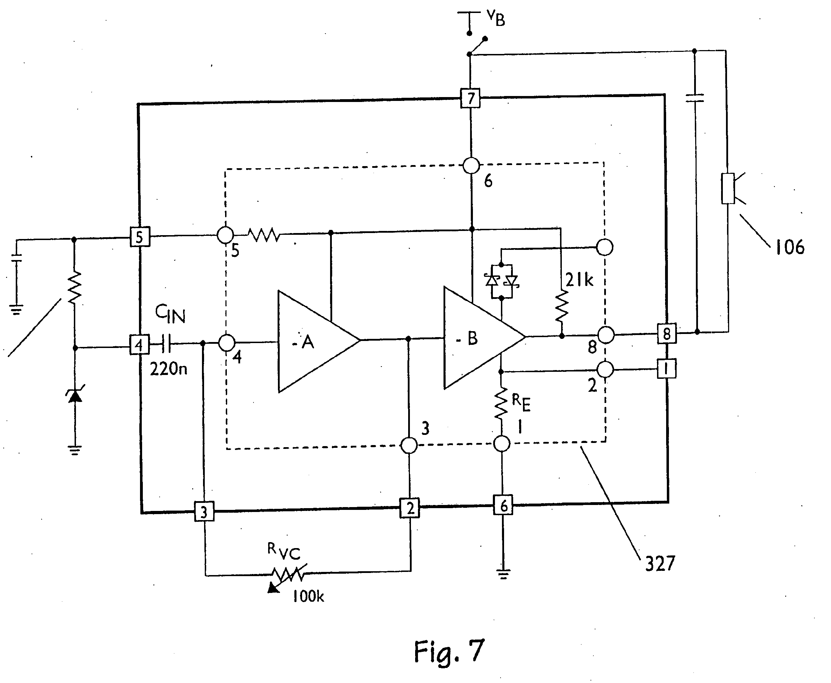

FIG. 7 shows a preferred embodiment of a two-stage Class A electrical circuit which has balanced stages for smoother voltage gain resulting in improved stability of signal and better battery life.

FIG. 8 shows a preferred embodiment of a three-stage Class D electrical circuit device combined with any of the shell models described herein or manufactured with an application-specific integrated circuit, where the amplifier input stage and the C-MOS output stage (class D) are populated on the same hybrid or on two separate hybrids. A basic operational amplifier 27 feeds into an inverting operational amplifier 128 forming the basic circuit. A Zener diode 29, Motorola type MLL4684 or equivalent, is set between ground potential and input into a capacitor 30 which establishes the corner frequency of the amplifier circuit. The noise generator formed by the Zener diode 29 and the RC input circuit requires very little current drain which is primarily dictated by the requirements of the Op Amp/Inverting Op Amp. The transducers are selected based on frequency response and impedance. Higher impedance transducers reduce the current draw. The total current drain required for the device is governed by the electrical / acoustic efficiency of the transducer and how much sound volume the device is set to deliver. The volume control 12 taper, as in the Class A design, is selected to have as much "bend" in it as possible. The reason is that most of the control occurs in the first few thousands of Ohms of resistance. The gain of the circuit is determined by a sum of the series gains of the subcircuits measured in decibels. So if the resistance selected on the volume control is minimum, gain is maximized to the loudest noise output. Resistance taper of the volume control yields about one and a half dB increments with a range typically of thirty-eight dB. In Figure 8, the receiver 306 could be a model FD 3286 or model FD 3287, commercially available from Cherry Electronics, Chicago, IL. Trimmer 130 is a 100K variable resistor.

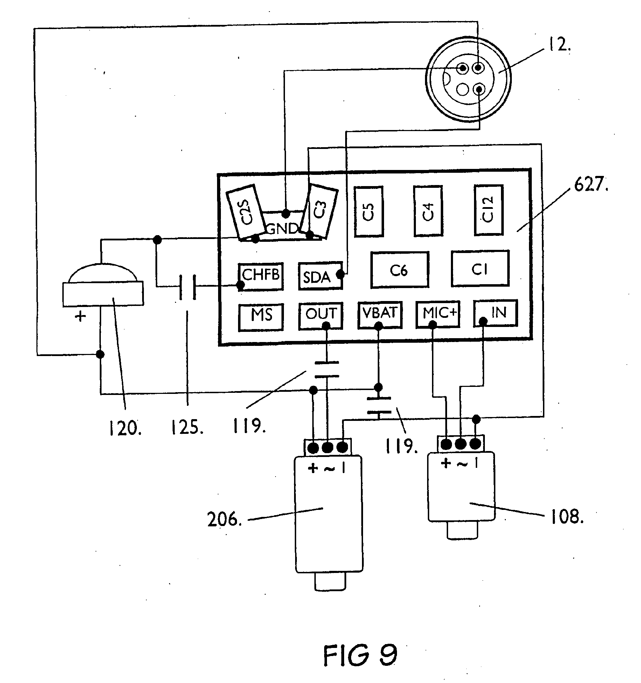

FIG. 9 shows a preferred embodiment of a programmable K-amp three-stage Class D electrical circuit. A suitable circuit for this purpose is shown in U.S. Patent No. 5,131,046.

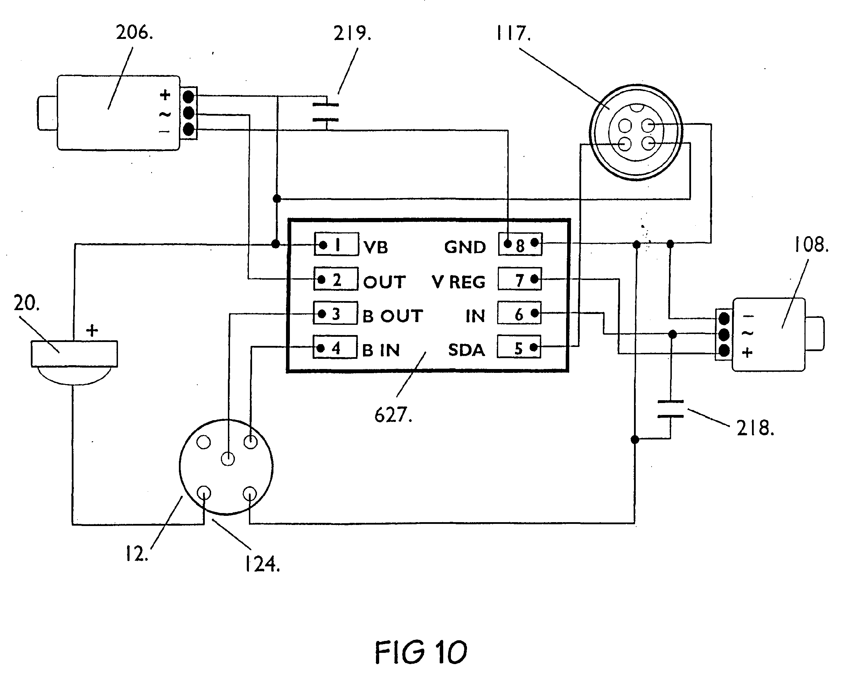

FIG. 10 shows a preferred embodiment of a programmable Intrigue™ three-stage, two-channel Class D electrical circuit.

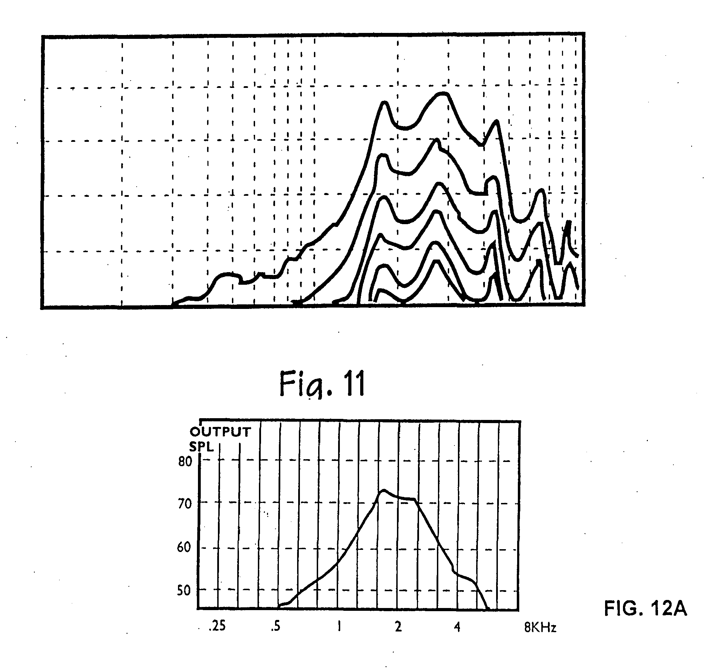

Figure 11 shows the frequency response of various volume control positions of the TRI 1, as measured on a Madsen Electronics, Inc. Model "Auricle" using a NOAH interface with a direct access 2 cc coupler. Table 1. shows the relationship of volume control rotation to noise gain. The taper of the volume control (VC) is set to give very discrete adjustment in the very low gain, barely audible ranges, with excellent voltage stability and a sound envelope of stable temporal characteristics, where head movement will not cause a change in the signal as measured at the TM 3. The VC was further designed to have a very quiet operation, free of scratchiness and changes in the consistency of taper during increase or decrease of gain.

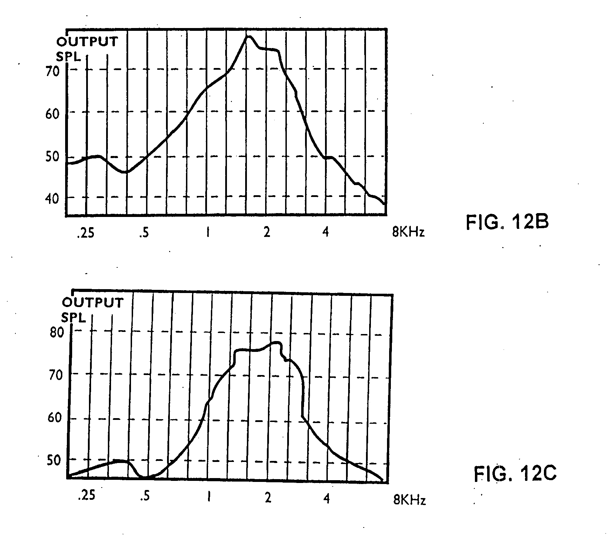

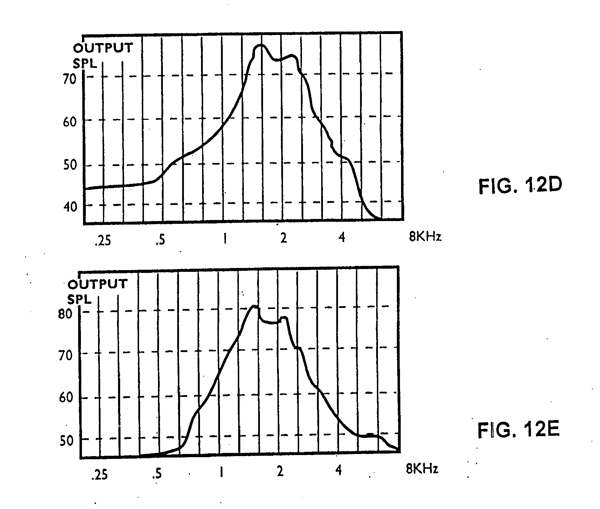

Figures 12A-12E show the spectrum characteristics of the intact noise stimulator which generates between an 82 dB and 85 dB noise level. The data is the average for nine devices.

### PARTS LIST:

The following is a list of elements used in the drawings and potential commercially available products which could be used as these elements:
1 COE shell
2 external ear canal
3 TM
4 inferior quadrant of the external ear canal
6 receiver
7 amplifier circuit
8 noise source circuit
9 face plate (such as an Entech 10A face plate)
10 battery door (such as an Entech 10A battery door)
11 battery compartment (e.g., an Entech 10A insulated battery compartment boot)
12 volume control (Microtronic, e.g.)
13 litz wires
14 concha retention arm
15 cavum retention arm
17 Helix Based OE-TRI device
18 CIC based TRI device
21 pina
22 extraction cord
23 acoustic horn
24 anterior ear canal
25 posterior ear canal
27 operational amplifier
28 transistor
29 Zener diode (such as a NTE Electronics Cat # 5005A Zener Diode)
30 capacitor (such as a Tansitor^{®} .068 microfarad capacitor)
33 vent retainer
34 vertical wall occupying coronal plane from superior to inferior to keep device 118 in place despite jaw action
35 footer to spread pressure of downward force of device 118 onto the inferior external canal across a sufficient surface area so as not to cause undue pressure on the superficial nerve endings of the external ear canal
106 Knowles^{®} ED 1914 receiver
108 Microphone (Knowles)
117 Microtronic variable resistor
118 alternative CIC based TRI device
119 output capacitor (Tansitor^{®})
120 1.1 V DC battery cell (typically #13, 312, 10A, and 5A from Rayovac)
124 Microtronic PJ 88 Volume Control
125 high frequency boost capacitor (Tansitor^{®})
131 on-board regulator
201 bias resistor (such as a Brill International 47 Ohm resistor)
202 feedback resistor for noise generator (such as a Brill International 10 k ohm resistor)
203 resistor (such as a Brill International 100 Ohm resistor)
206 Receiver (Knowles)
218 output capacitor
219 output isolation capacitor (Tansitor^{®}) (duplication of Vbatt)
227 Monolithic IC High Gain Class A Amplifier
327 Monolithic IC (Two stage) High Gain Class A Amplifier
395 Tansitor^{®} 2.2 microfarad capacitor
397 Tansitor^{®} .047 microfarad capacitor
427 Monolithic IC (Two stage) High Gain Class D Amplifier
527 C-MOS amplifier, output stage Class D
627 K-amp programmable integrated circuit, commercially available from Etymotic Research
C_{IN} 220 nano farad capacitor

| **Table 1. Volume control position to output noise level** | | |
|---|---|---|
| **VC Set** | **Gain** | **Peak Frequency** |
| On | -5 dB | 10,000 Hz |
| 1/8 On | -4 dB | 2727 Hz |
| 2/8 On | 0 dB | 2720 Hz |
| 3/8 On | 10 dB | 2720 Hz |
| ½ On | 13 dB | 2720 Hz |
| 5/8 On | 16 dB | 2800 Hz |
| ¾ On | 17 dB | 2800 Hz |
| 7/8 On | 19 dB | 2720 Hz |
| 7.5/8 On | 25 dB | 2720 Hz |
| Full-on | 38 dB | 2900 Hz |
| | | |

All measurements disclosed herein are at standard temperature and pressure, at sea level on Earth, unless indicated otherwise. All materials used or intended to be used in a human being are biocompatible, unless indicated otherwise.

The foregoing embodiments are presented by way of example only; the scope of the present invention is to be limited only by the following claims.

## Claims

1. An in-the-ear device for treating tinnitus, said device comprising :
- a housing (1) which is configured to fit within an ear canal (2) of a user, said ear canal (2) including a tympanic membrane (3),
- a sound generator within said housing (1) for generating sounds to habituate said tinnitus, and
- a receiver (6) within said ear canal (2) for delivering said sounds from said sound generator to said user so that said tympanic membrane (3) is deflected by said sounds,
**characterized in that** said housing can be positioned within the car canal (2) so that the ear canal (2) is at least partially open to receive sounds from surrounding ambient sources,
said device further comprising retention arms or a vent retainer, for positioning said housing (1) within said ear canal (2) so that said ear canal (2) is at least partially open to receive sounds from surrounding ambient sources.

2. The in-the-ear device according to claim 1, wherein said sound generator is configured to provide a wide band sound spectrum to emulate real world sound.

3. The in-the-ear device according to claim 1, wherein said sound generator is configured to generate sound of stable temporal characteristics so that user head movement does not cause any change in signal at the user's tympanic membrane (3).

4. The in-the-ear device according to claim 1, wherein said sound generator comprises a noise generator and said positioning means is configured to aim said receiver at the superior portion of the user's tympanic membrane.

5. The in-the-ear device according to claim 4, wherein said sound generator comprises a noise source (8), an amplifier (7) connected to said noise source (8), and said receiver (6) connected to said amplifier (7), and a horn (13) within said housing connected to said receiver (6).

6. The in-the-ear device according to claim 5, wherein said noise generating means further comprises means for adjusting noise level.

7. The in-the-ear device according to claim 6, further comprising an extraction cord attached to said housing and said extraction cord is configured to serve as an extension for said noise level adjusting means.

8. The in-the-ear device according to claim 1, further comprising a battery located within said housing.

9. The in-the ear device according to claim 1, comprising upper and lower retention arms (14, 15) connected to said housing (1).

10. The in-the-ear device according to claim 8, comprising a medial ipsolateral-route-of-signal vent retainer (33) attached to a distal end of said device, and wherein said retainer is configured to support the device upward into the superior portion of the external ear canal (2) while keeping the interior canal open and substantially unobstructed for free-field sound transmission.

11. The in-the-ear device according to claim 10, further comprising said housing having a face plate (9) and said face plate (9) having an opening at the apex of the external ear canal (2).

12. The in-the-ear device according to claim 1, wherein said sound generator comprises a programmable electric circuit.

13. The in-the-ear device according to claim 1, wherein said sound generator comprises a subminiature electronic noise-generator means for generating noise within an external auditory canal (2) of a user of the device and means for adjusting the volume of the noise to habituate the user to tinnitus head/ear noise.

14. The in-the-ear device according to claim 1, wherein said sound generator is programmable.

15. The in-the-ear device according to claim 1, further comprising a hearing aid for amplifying ambient sounds.

16. The in-the-ear device according to claim 1, further comprising said device being an open ear helix based device.

17. The in-the-ear device according to claim 1, wherein said device is further configured to treat hyperacusis.

18. The in-the-ear device according to claim 1, wherein said housing has an outer dimension which leaves said ear canal (2) at least partially open to receive sounds from surrounding ambient sources when said housing is positioned within said ear canal.

## Patentansprüche

1. In-dem-Ohr-Gerät zur Behandlung von Tinnitus, wobei das Gerät Folgendes umfasst:
- ein Gehäuse (1), das dazu konfiguriert ist, in einen Gehörgang (2) eines Benutzers zu passen, wobei der Gehörgang (2) ein Trommelfell (3) enthält,
- einen Tongenerator in dem Gehäuse (1) zur Erzeugung von Tönen zur Gewöhnung an Tinnitus, und
- einen Empfänger (6) in dem Gehörgang (2) zur Zuführung der Töne von dem Tongenerator zu dem Benutzer, so dass das Trommelfell (3) durch die Töne ausgelenkt wird, **dadurch gekennzeichnet, dass** das Gehäuse so in dem Gehörgang (2) positioniert sein kann, dass der Gehörgang (2) für den Empfang von Tönen von umliegenden Umgebungsquellen zumindest teilweise offen ist, wobei die Vorrichtung ferner Haltearme oder einen Vent-Halter zur Positionierung des Gehäuses (1) in dem Gehörgang (2) derart, dass der Gehörgang (2) für den Empfang von Tönen von umliegenden Umgebungsquellen zumindest teilweise offen ist, umfasst.

2. In-dem-Ohr-Gerät nach Anspruch 1, wobei der Tongenerator dazu konfiguriert ist, ein Breitbandklangspektrum bereitzustellen, um reale Töne zu emulieren.

3. In-dem-Ohr-Gerät nach Anspruch 1, wobei der Tongenerator dazu konfiguriert ist, Töne mit stabilen zeitbezogenen Charakteristika zu erzeugen, so dass eine Kopfbewegung des Benutzers keine Signaländerungen am Trommelfell (3) des Benutzers verursacht.

4. In-dem-Ohr-Gerät nach Anspruch 1, wobei der Tongenerator einen Geräuschgenerator umfasst und das Positioniermittel dazu konfiguriert ist, den Empfänger auf den oberen Teil des Trommelfells des Benutzers auszurichten.

5. In-dem-Ohr-Gerät nach Anspruch 4, wobei der Tongenerator eine Geräuschquelle (8), einen mit der Geräuschquelle (8) verbundenen Verstärker (7) und den mit dem Verstärker (7) verbundenen Empfänger (6) sowie einen Schalltrichter (13) in dem Gehäuse, der mit dem Empfänger (6) verbunden ist, umfasst.

6. In-dem-Ohr-Gerät nach Anspruch 5, wobei das Geräuscherzeugungsmittel ferner ein Mittel zur Einstellung des Geräuschpegels umfasst.

7. In-dem-Ohr-Gerät nach Anspruch 6, das ferner eine an dem Gehäuse befestigte Herausziehkordel umfasst, wobei die Herausziehkordel dazu konfiguriert ist, als Verlängerung für das Mittel zur Einstellung des Geräuschpegels zu dienen.

8. In-dem-Ohr-Gerät nach Anspruch 1, das ferner eine in dem Gehäuse positionierte Batterie umfasst.

9. In-dem-Ohr-Gerät nach Anspruch 1, das ferner einen oberen und einen unteren mit dem Gehäuse (1) verbundenen Haltearm (14, 15) umfasst.

10. In-dem-Ohr-Gerät nach Anspruch 8, das einen mittleren IROS-Vent-Halter (33) umfasst, der an einem distalen Ende des Geräts befestigt ist, und wobei der Halter dazu konfiguriert ist, das Gerät nach oben hin in den oberen Teil des äußeren Gehörgangs (2) zu stützen, während der innere Gehörgang offen und für Freifeld-Tonübertragung im Wesentlichen unversperrt gehalten wird.

11. In-dem-Ohr-Gerät nach Anspruch 10, das ferner umfasst, dass das Gehäuse eine Abschlussplatte (9) aufweist und die Abschlussplatte (9) eine Öffnung an der Spitze des äußeren Gehörgangs (2) aufweist.

12. In-dem-Ohr-Gerät nach Anspruch 1, wobei der Tongenerator eine programmierbare elektrische Schaltung umfasst.

13. In-dem-Ohr-Gerät nach Anspruch 1, wobei der Tongenerator ein elektronisches Subminiaturgeräuschgeneratormittel zur Erzeugung von Geräuschen in einem äußeren Gehörgang (2) eines Benutzers des Geräts und ein Mittel zur Einstellung der Lautstärke der Geräusche zum Gewöhnen des Benutzers an Tinnituskopf-/-ohrgeräusche umfasst.

14. In-dem-Ohr-Gerät nach Anspruch 1, wobei der Tongenerator programmierbar ist.

15. In-dem-Ohr-Gerät nach Anspruch 1, das weiterhin eine Hörhilfe zur Verstärkung von Umgebungsgeräuschen umfasst.

16. In-dem-Ohr-Gerät nach Anspruch 1, das weiterhin umfasst, dass das Gerät ein offenes-Ohr-Gerät auf Helixbasis ist.

17. In-dem-Ohr-Gerät nach Anspruch 1, wobei das Gerät ferner dazu konfiguriert ist, Hyperakusis zu behandeln.

18. In-dem-Ohr-Gerät nach Anspruch 1, wobei das Gehäuse ein Außenmaß hat, das den Gehörgang (2) für den Empfang von Tönen von umliegenden Umgebungsquellen zumindest teilweise offen lässt, wenn das Gehäuse in dem Gehörgang positioniert ist.

## Revendications

1. Dispositif intra-auriculaire destiné au traitement de l'acouphène, ledit dispositif comportant :
- un boîtier (1) qui est configuré pour s'ajuster à l'intérieur d'un conduit auditif (2) d'un utilisateur, ledit conduit auditif (2) comprenant une membrane tympanique (3),
- un générateur de sons à l'intérieur de ledit boîtier (1) servant à générer des sons pour accoutumer ledit acouphène, et
- un récepteur (6) à l'intérieur dudit conduit auditif (2) servant à amener lesdits sons dudit générateur de sons audit utilisateur de telle sorte que ladite membrane tympanique (3) soit déformée par lesdits sons, **caractérisé en ce que** ledit boîtier peut être positionné à l'intérieur du conduit auditif (2) de telle sorte que le conduit auditif (2) soit au moins partiellement ouvert pour recevoir des sons en provenance de sources ambiantes environnantes, ledit dispositif comportant en outre des bras de retenue ou un ancrage d'évent, servant à positionner ledit boîtier (1) à l'intérieur dudit conduit auditif (2) de telle sorte que ledit conduit auditif (2) soit au moins partiellement ouvert pour recevoir des sons en provenance de sources ambiantes environnantes.

2. Dispositif intra-auriculaire selon la revendication 1, ledit générateur de sons étant configuré pour émettre un spectre sonore à large bande afin d'émuler le son du monde réel.

3. Dispositif intra-auriculaire selon la revendication 1, ledit générateur de sons étant configuré pour générer un son de caractéristiques temporelles stables de telle sorte qu'un mouvement de tête de l'utilisateur ne provoque aucun changement du signal au niveau de la membrane tympanique (3) de l'utilisateur.

4. Dispositif intra-auriculaire selon la revendication 1, ledit générateur de sons comportant un générateur de bruit et ledit moyen de positionnement étant configuré pour pointer ledit récepteur vers la partie supérieure de la membrane tympanique de l'utilisateur.

5. Dispositif intra-auriculaire selon la revendication 4, ledit générateur de sons comportant une source (8) de bruit, un amplificateur (7) relié à ladite source (8) de bruit, ledit récepteur (6) relié audit amplificateur (7), et un cornet (13) à l'intérieur dudit boîtier relié audit récepteur (6).

6. Dispositif intra-auriculaire selon la revendication 5, ledit moyen générateur de bruit comportant en outre un moyen de réglage du niveau de bruit.

7. Dispositif intra-auriculaire selon la revendication 6, comportant en outre un cordon d'extraction fixé audit boîtier et ledit cordon d'extraction étant configuré pour servir de prolongement audit moyen de réglage du niveau de bruit.

8. Dispositif intra-auriculaire selon la revendication 1, comportant en outre une batterie située à l'intérieur dudit boîtier.

9. Dispositif intra-auriculaire selon la revendication 1, comportant des bras supérieur et inférieur (14, 15) de retenue reliés audit boîtier (1).

10. Dispositif intra-auriculaire selon la revendication 8, comportant un ancrage (33) d'évent médian homolatéral au parcours du signal et fixé à une extrémité distale dudit dispositif, et ledit ancrage étant configuré pour soutenir le dispositif vers le haut jusque dans la partie supérieure du conduit auditif externe (2) tout en maintenant le canal intérieur ouvert et sensiblement dégagé pour une transmission du son en champ libre.

11. Dispositif intra-auriculaire selon la revendication 10, **caractérisé en outre en ce que** ledit boîtier est doté d'une plaque (9) de face et **en ce que** ladite plaque (9) de face présente une ouverture en tête du conduit auditif externe (2).

12. Dispositif intra-auriculaire selon la revendication 1, ledit générateur de sons comportant un circuit électrique programmable.

13. Dispositif intra-auriculaire selon la revendication 1, ledit générateur de sons comportant un moyen électronique sous-miniature générateur de bruit servant à générer du bruit à l'intérieur d'un canal auditif externe (2) d'un utilisateur du dispositif et un moyen de réglage du volume du bruit pour accoutumer l'utilisateur à un bruit tête / oreille d'acouphène.

14. Dispositif intra-auriculaire selon la revendication 1, ledit générateur de sons étant programmable.

15. Dispositif intra-auriculaire selon la revendication 1, comportant en outre une prothèse auditive servant à amplifier des sons ambiants.

16. Dispositif intra-auriculaire selon la revendication 1, **caractérisé en outre en ce que** ledit dispositif est un dispositif à oreille ouverte basé sur l'hélix.

17. Dispositif intra-auriculaire selon la revendication 1, ledit dispositif étant en outre configuré pour traiter l'hyperacousie.

18. Dispositif intra-auriculaire selon la revendication 1, ledit boîtier présentant une dimension extérieure qui laisse ledit conduit auditif (2) au moins partiellement ouvert pour recevoir des sons en provenance de sources ambiantes environnantes lorsque ledit boîtier est positionné à l'intérieur dudit conduit auditif.
